# EUROPEAN PATENT APPLICATION

(11) **EP 1 174 034 A1**
(43) Date of publication of application: **23.01.2002**
(21) Application number: 01117089.1
(22) Date of filing: 13.07.2001
(51) Int. Cl.: A22B 5/00

(54) **Method for trimming pork bellies**

(30) Priority: 19.07.2000 US 619562
(71) Applicant: FMC, Chicago, IL 60601 (US)
(72) Inventor: Vogeley, Arthur W., Jr., Seattle, WA 98112 (US); Larreau, Bret J., Berlin-Heights, OH 44814 (US); Wiesinger, Erich W.A., Issaquah, WA 98027 (US); Van Liew, Gregory S., Seattle, WA 98122 (US)
(74) Representative: Thylén, Eva Matilda

(57) **Abstract**

A method for portioning hog carcass sides is disclosed. In one use of the invention, the hog carcass is portioned according to a computer generated scribe line. The scribe line is generated by mapping the endpoints of lean lines of meat. The method contemplates generating a scribe line without thickness mapping of the actual scribe line. The invention may be used in hog butchering processes where industry standards mandate portioning of the fatback be made with relation to the scribe line. Trimming of the belly can be carried out using the computer generated scribe line as well.

## Description

### Field of the Invention

This invention relates to the meat processing industry, and more particularly to methods for use in trimming pork bellies.

### Background of the Invention

Slaughterhouses portion hog carcasses into two similar halves by cutting down the middle of the backbone. The hog halves are then further portioned into primal cuts including the foot (front and back), fatback, loin, belly, shoulder, ham and jowl. Ham and shoulder cuts are generally removed before sectioning the midsection of the hog. The midsection contains the fatback, loin, ribs and belly. A pork belly is the cut of meat taken from a hog half midsection after removal of the loin, fatback, and ribs. Pork bellies are required to be trimmed of fat and defects. The ham includes the leg and hind quarters of the animal. The shoulder is the anterior portion of the animal including the butt and picnic. Usually, a next step is to portion the hog midsection into its respective primal cuts.

There are numerous patents which describe methods and apparatus for removing the various cuts from the midsection portion of the hog. For example, in U.S. Patent No. 5,902,177 to Tessier et al., a method and apparatus are described which determines the thickness of the ribs and then derives a cut profile according to a predetermined meat thickness which is desirable to leave under the ribs. U.S Patent No. 4,662,029 to Helsene et al., also describes a method of separating the loin and removing the ribs from the pork half. In this method, after the loin and ribs are removed, the fatback is removed with a fatback knife that makes a cut three inches from the longitudinal edge closest to the backbone side. The position of the fatback knife is controlled by the computer in response to information conveyed by a vision system camera. The bellies are further trimmed in response to the information read and transmitted by the vision system camera.

Using either of the aforementioned methods to remove the ribs, the hog half is left with a region of meat where the ribs have been removed and thicker portions where there were no ribs. Underlying the ribs is a network of lean lines of meat interspersed with fat (the "fingers of lean"). Since ribs do not cover all of the surface area of the pork belly, there is a curved line remaining after separation of the ribs from the belly where the ribs were attached to the loin. The demarcation line between the two areas is often referred to as the scribe line. The ends of the lean fingers correlate substantially to the location of the scribe line. This is common in all hogs. Thus, a scribe line is generally suited to serve as a reference for trimming pork bellies to a proportional standard for all hog species.

Beef associations, such as the National Association of Meat Purveyors (NAMP), set voluntary standards for the various cuts of meat. Those producers which choose to follow the standards set forth by these associations made up of industry participants earn the right to advertise their compliance with the associations' standards. Some standards may exceed those set by the United States Department of Agriculture. Thus, those meat processors which follow the standards set forth by a self-regulating segment of the industry will be able to command higher prices for their meat products. NAMP has set forth a certain standard for pork bellies which uses the scribe line as the reference for trimming the pork bellies. For example, NAMP mandates that the fatback be removed from the belly by a straight cut not more than 1½ inches from the outermost dorsal curvature of the scribe line. Further trimming of the pork belly likewise references the scribe line to comply with another standard for removal of scores and snowballs that measure in excess of 3 inches square. This standard specifies that the scores and snowballs be removed from the ventral side of the scribe line. The scribe line will not be considered a score so long as it is less that ¼ of an inch at any point. Thus, it becomes important to have an accurate measure of the scribe line to meet the standards.

Automated means of trimming pork bellies are now in existence which use video cameras or other optical devices to guide the cutting apparatus. The video cameras reference the trimming that is to be performed on the pork belly from the scribe line as mandated by the standards. There are devices which are able to discern areas of thick and thin meat regions and which can produce a thickness map of the meat portion. See for example U.S. Patent No. 5,324,228 to Vogeley, Jr. and more specifically, the application it references, Serial No. 07/772,309. However, the scribe line may often be difficult to discern because industry standards also mandate the depth of the scribe line, and on a flat surface, the scribe line can distort causing both thin and thick areas to appear equal in proportion. If cut too deep, the scribe line is seen as an undesirable score on the meat, which will lead to rejection of the product. Also, if a cut is too shallow, the methods for detecting areas of thin and thick regions do not accurately reflect the true scribe line. This, coupled with the fact that most trimming is done on a flat surface, makes scanning and determining the scribe line difficult. Over time, errors in trimming due to faulty prediction of the scribe line may cost the industry vast sums of money and time. In addition, middle distributors, and eventually the consumers, are not guaranteed a consistent quality product.

Prior methods of trimming use video or optical cameras that are able to detect differences in the surface contours to generate thickness maps of the meat products. However, the narrow tolerances between having an acceptable scribe line and overscoring the meat prevents most of these methods from being used efficiently to determine exactly where the scribe line lies.

Therefore, there is a longstanding need for a more reliable method of ascertaining a suitable reference, such as a scribe line, to be used in the meat industry. The method preferably works with existing devices, such as video and other optical cameras to properly guide cutting devices in trimming pork bellies and other meat products.

### Summary of the Invention

The present invention is directed to a method of trimming pork bellies using a method of calculating the location of the scribe line by measuring the ends of lean lines ("fingers") with the use of the present video imaging technology as opposed to previous methods of direct contouring or thickness mapping. The method of the invention includes the steps of scanning the meat portion in a manner that is able to discern the lean tissue from fat tissue. Then analyzing the image to determine the ends of the lean "fingers". Connecting the coordinates of the ends of the lean "fingers" to build a continuous reference line gives a reference related to the location of the scribe line. Finally, the meat portion is trimmed using the computer generated line as opposed to a scribe line obtained from thickness mapping.

In a preferred embodiment, the method is used in a process for portioning a side of a hog carcass. The side includes primal cuts of meat such as the ham, shoulder, fatback, loin and belly. A step in the method includes removing the ham and shoulder primals and leaving the fatback, loin and belly as one midsection unit. A next step is removing the ribs leaving behind a midsection which includes thin and thick regions of meat. Underlying the ribs is a network of lean meat interspersed with fat. The ends of the lean lines accurately references the scribe line. A computer having a scanning device can generate a scribe line from the ends of the lean tissue accurately representing the actual scribe line. The computer generated scribe line is used to portion the fatback from the midsection. The computer generated scribe line is also used as a reference in trimming the belly of defects.

Use of the existing technology which is capable of discerning between areas of lean and fat tissue may be advantageously used in this method to generate an accurate scribe line to use in referencing any trimming operations for any of the various cuts of meats.

### Brief Description of the Drawings

The foregoing aspects and many of the attendant advantages of this invention will become more readily appreciated as the same become better understood by reference to the following detailed description, when taken in conjunction with the accompanying drawings, wherein:
FIGURE 1 is a schematic representation of a hog carcass showing the primal cuts of meat
FIGURE 2 is a plan side view of a hog midsection showing the fatback, loin, belly and ribs
FIGURE 3 is a plan side view of the hog midsection of FIGURE 3 without the ribs
FIGURE 4 is a cross-sectional view of the hog midsection of FIGURE 4 showing the thin and thick meat portions after removal of the ribs
FIGURE 5 is a schematic representation of the apparatus used to carry out the method of the present invention
FIGURE 6 is a schematic representation of a method in accordance with the present invention.

### Detailed Description of the Preferred Embodiment

The present invention is directed to a method of trimming primal cuts of meat using scanners which can detect a discontinuity in tissue, such as occurs between fat, bone, or cartilage with lean tissue. The method uses video or optical scanning devices to determine a plurality of termini of one of the tissue types, such as the lean tissue, bone, fat or cartilage and then uses those termini to build a suitable reference line with the aid of a computer. The computer then directs further processing or trimming of the meat with reference to that line. The-method of the present invention can be used in any instance whenever termini of any tissue type signifies the appropriate reference line to be used in trimming. Thus, the present invention may be used in any of a number of meat processing steps which rely on first locating a suitable reference line and then guiding a cutting device in accordance with that reference line. The preferred embodiment will be described with reference to hog butchering, however, the method of the present invention is adaptable to any meat portioning operation, provided there are tissues with termini correlating to a reference.

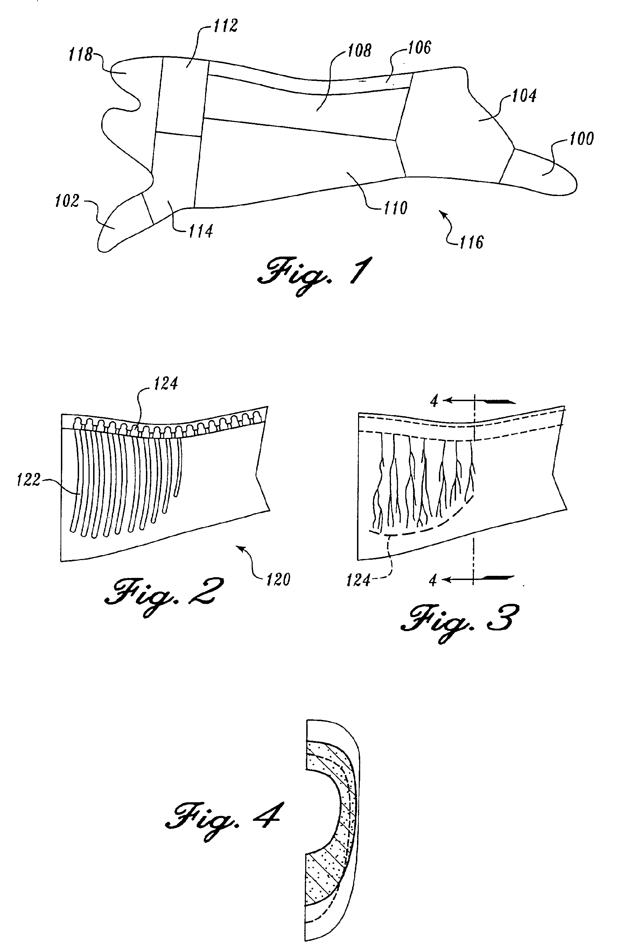

In a typical method of butchering hogs, the hog is first sectioned into two halves by cutting the hog down the middle of the backbone. The carcass now represents two distinct but similar sides. Each side portion of the hog is further subdivided into primals as shown in FIGURE 1. The hog side includes the hind foot 100 and forefoot 102, the ham 104, fatback 106, loin 108, belly 110, butt 112 and picnic 114. Collectively, the butt 112 and picnic 114 are referred to as the shoulder. Processing of the hog sides into primals continues identically for the two sides. Helsene et al. (U.S. Patent No. 4,662,029) and Vogt et al. (U.S. Patent No. 3,159,869) provide a general explanation of the sequence of hog butchering steps.

In a typical hog slaughterhouse, the ham 104 including the hind foot 100, as well as the shoulder 116, including the forefoot 102 and jowl 118, are removed prior to sectioning the midsection primals. The shoulder 116 may further be sectioned into the butt 112, picnic 114 and jowl 118. The midsection, generally denoted as 120, includes the fatback 106, lying in the dorsalmost part of the hog carcass. Ventrally to the fatback 106 lies the loin 108. The loin contains the longissimus dorsi muscle which forms the major paraspinal muscle. The loin may further be divided into rib chops, centerloin chops and tenderloin chops, from front to back, respectively. Lying ventrally of the loin is the belly 110. The midsection also includes ribs 122 and a portion of the backbone 124.

In a typical hog processing plant, the ribs 122 are removed prior to sectioning the midsection 120 into the three primals discussed above. A suitable method for removing the ribs 122 is described in Helsene et al., which uses a knife to section the ribs from the midsection with guidance from a vision system cameral. While Helsene et al. describes an adequate method of removing ribs, the method still relies in large part on manual interaction and is thus not fully automated as in another method described in U.S. Patent No. 5,902,177 to Tessier et al., which is herein incorporated by reference. In Tessier et al., an optical imaging device including a laser and camera is able to determine the thickness of the ribs using optical triangulation techniques. The computer then performs calculations to determine the appropriate depth to trim the ribs. The meat underlying the ribs is filled with lean lines interspersed with fat. The ventral terminal ends of the lines follow a path related to the location of the scribe line.

Once the ribs 122 have been removed, the hog midsection is ready to be portioned into primal cuts including the fatback 106, loin 108 and belly 110. The Helsene et al. '029 patent and the Vogt '869 patent, both of which are herein incorporated by reference, describe ways of trimming the loin from the midsection. Vogt et al. uses a semi-automatic method consisting of a movably mounted table and U-shaped cutting knife. The loin removal step can be accompanied with removal of the backbone. Helsene similarly uses a U-shaped knife to remove the loin from the midsection.

Once the ribs and loin have been removed, the belly and fatback primals remain attached to one another. The fatback 106 can be trimmed from the loin 108 as described in Helsene et al. In Helesene, a fatback knife cuts a 3-inch strip from the belly along a longitudinal edge which is the closest to the backbone. The position of the fatback knife is controlled by a computer in response to the information conveyed by the vision system camera. Vogt et al. also describes a method of removing the fatback in Application Serial No. 203,931.

It should be readily apparent that the ordering of steps is no consequence to the present invention, therefore in some embodiments, the loin removal step may precede the rib removal step, or the fatback removal step may precede both the rib and loin removal steps or any combination thereof. These embodiments are meant to be included within the scope of the present invention and the invention can be advantageously used in these steps as well. Equally suitable is the use of the present invention in other meat processing industries such as fowl, fish or cattle.

The present invention is meant to be used in any meat portioning operation, including those described above, or those not mentioned but well known to others in fowl, fish or beef portioning industries.

The preferred embodiment of the present invention contemplates using the termini of lean lines of meat underlying the ribs to construct a reference line which may be used in trimming the meat portion according to that line. Furthermore, the present invention can be used with scanners and automated cutting devices already in existence which reduces the cost of converting old processes into the present invention. And furthermore, other tissue types such as bone or cartilage may be used, so long as the termini of the tissue signifies a desirable reference line. Because the present invention relies on the inherent properties of the meat, there is no scoring or thickness mapping that is required to take place in order to build the reference line. This is a significant difference between the present invention and previous methods of portioning meat, especially in trimming pork bellies.

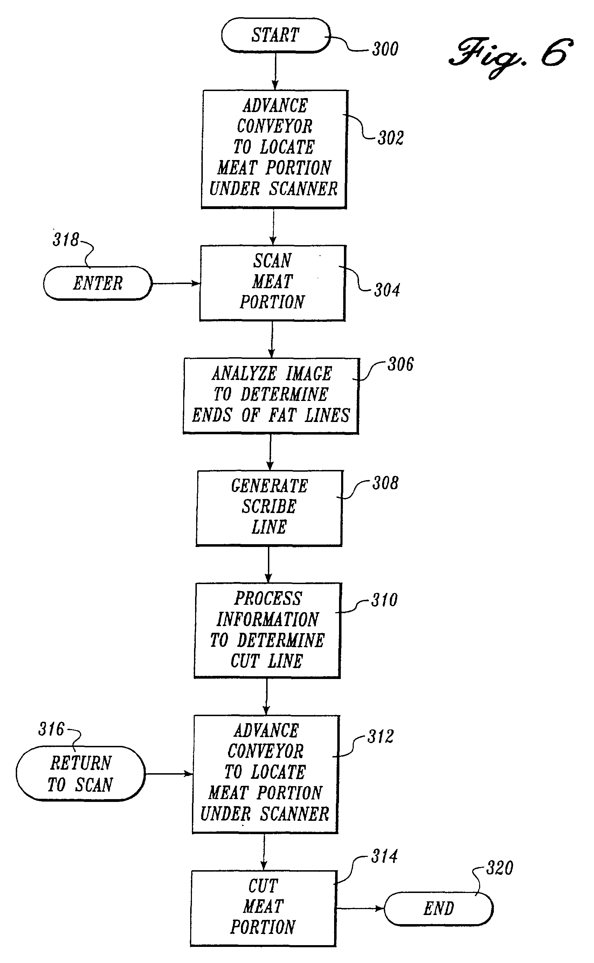

As shown schematically in FIGURE 6, the first step 302 is to advance the conveyor carrying a meat portion to the scanning station where the meat portion is scanned in step 302.

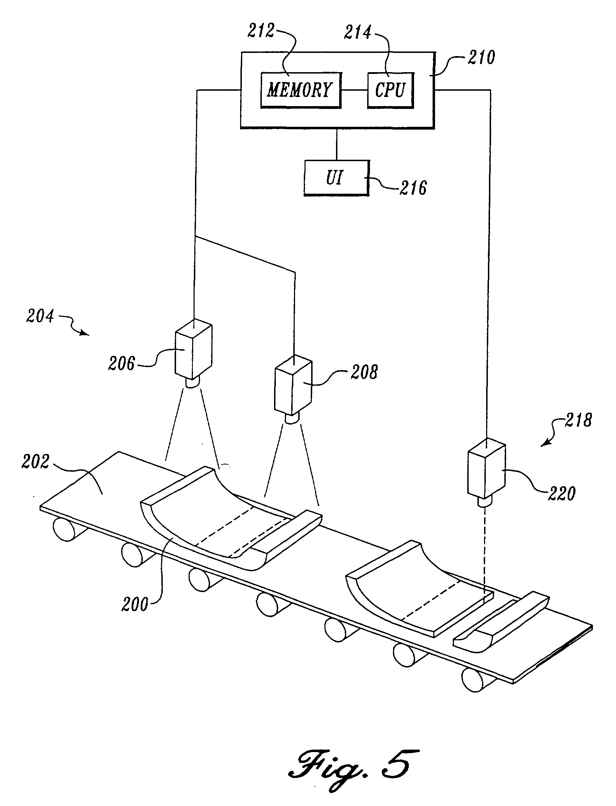

As shown in FIGURE 5, in the preferred embodiment of the present invention, the hog carcass 200 is carried on a endless conveyor mechanism 202 which moves the hog carcass 200 through a series of stations. The movement of the conveyor system 202 may be continuous or may be in discrete steps, i.e., stopping at each station. A combination of movable overhead devices and a continuously moving conveyor system may provide the advantage of increasing productivity. In accordance with the present invention, the hog carcass 200 passes through a scanning system, generally denoted by 204. The scanning system 204 is able to detect regions of fat and lean muscle tissue and, more particularly, the scanning system is able to discern the ends of lean lines or "fingers". The lean lines underlie the ribs and are visible once the ribs have been removed in a previous processing operation. Suitable scanning systems are described in U.S. Patent Nos. 5,324,228 to Vogeley, Jr. and 5,937,080 to Vogeley, Jr. et al., which are herein incorporated by reference. The scanning system 204 may use one or a plurality of cameras 206, 208. Furthermore, the scanning devices 206, 208 need not use electromagnetic radiation in the visible light region, other scanning devices which use higher or lower frequency radiation may be used as well, for example X-rays or infrared radiation or any other suitable energy wave in the electromagnetic radiation spectrum. The scanning devices 206, 208 work on the principle that different kinds of tissue effect electromagnetic waves in different ways. For example, fat and lean tissue are made of different components, or even if they have similar components, one tissue may have a higher or lower amount of the same component. Scanners rely on the inherent differences between tissues to measure the effects the tissues have on the radiation, such as the amount that is reflected. Sensors mounted in strategic positions can record the reflectance or transmittance of the radiation, and provide data which is further analyzed to determine the boundaries between tissue types.

Following the scanning step 304 of FIGURE 6, steps for analyzing the data, generating a scribe line, and processing the information to determine the cutting path can proceed, respectively in steps 306, 308 and 310, according to FIGURE 6.

As shown in FIGURE 5, the preferred embodiment to carry out the above steps will include a computer 210 having a memory 212, a central processing unit 214 (CPU), and a user interface 216. Collectively, these devices will store, analyze and process the data collected from the scanners 206, 208. Not only will the computer store and analyze the data, but the computer may also network or interface with other computers running different parts of the butchering system, such as earlier primal removing steps, or the computer may drive peripherals such as the conveyor, the scanners or the cutting devices. Between computers, they may send signals or flags to trigger one event, provided completion of a preliminary step has occurred. In this manner, computers can provide checks for one another to enable a completely automated system. The memory 212 can store instructions in the form of a computer program which executes upon the happening of a given event, such as input from a user or from a different computer or any peripheral devices. The data entering via the scanning devices may likewise be stored in the computer memory. For instance, an event may alert the scanner that a food portion has entered within the scope of the scanners and data needs to be collected. The data sent to the computer can be collected and stored to await further processing. When all the data for the given foodstuff has been collected, an event will be triggered to end data collection and signal that the next scan will be for a different food portion. The data is now processed and analyzed.

In the method according to the present invention, the data will be useful in determining the regions of fat and lean tissue. FIGURE 2 shows a side of a hog carcass including the ribs, backbone, fatback, loin and belly. FIGURE 3 shows the same side of the hog carcass with the ribs and backbone removed and FIGURE 4 shows the cross section of FIGURE 3. In a hog carcass, the tissue of interest for purposes of the present invention is in the form of alternating lean and fat lines which underlie the ribs. The ends of these lines substantially correspond to the scribe line 124 of FIGURE 3 which marks the division between the thinner regions of meat where the ribs were attached and the thicker regions of meat. The CPU 214 is able to analyze the data and determine therefrom the termini or endpoints of the lean tissue which substantially correlates to the scribe line 124 according to a pre-programmed algorithm. The CPU 214 can assign coordinates to the endpoints of the lean lines. Once the endpoints have been assigned coordinates, the CPU 214 may generate a scribe line which is substantially an accurate representation of the actual scribe line. In fact, the computer generated scribe line may be closer to the ideal scribe line because it uses the anatomical features rather than a cut line that is prone to have errors. Thus, the method of the present invention can use well-know scanning devices in a way to advantageously derive a computer-generated scribe line which is not dependent on the thickness measurement the way previous methods used to. The method of the present invention is preferable to methods which rely on contour or thickness mapping the surface of the hog carcass. This is a significant difference between the present invention and previous methods.

A step in the preferred method of the present invention uses the computer generated scribe line as a reference in trimming the midsection of the hog carcass, shown as step 314 in FIGURE 6. One trimming operation where the scribe line is a useful reference is the fatback removal step. The fatback is the part of the hog carcass that lies dorsally to all other parts. The fatback is a relatively continuous section of fat tissue. The computer generated scribe line is the reference used to remove the fatback rather than the actual scribe line which may be displaced a considerable distance from the ideal scribe line due to previous errors in the de-ribbing operation. Since the present invention relies on the ideal scribe line, better results are obtained since the error in previous steps is not carried over into this step. One standard adopted throughout the industry and by NAMP is that the fatback shall be removed by a straight cut not more than 1½ inches from the outermost dorsal curvature of the scribe line. Thus, the computer generated scribe line can be used to measure this exact amount of tissue to trim off. Furthermore, other standards regulate the amount and size of defects which lie ventrally of the scribe line. Similarly, the present invention provides a computer generated scribe line which can be used to comply with this standard as well.

Step 314, the trimming of the fatback, can take place in a downstream station generally denoted as 218 in FIGURE 5. At the same time the conveyor is continually advancing to a predetermined location to allow the cutting device to perform the cutting step 314, the conveyor places another hog carcass underneath the scanner and begins the method again from step 312 for another hog carcass and returns to step 304 as shown in FIGURE 6. In step 314, the computer generates the appropriate cutting path by a pre-programmed algorithm to meet specific standards, such as described above, and then directs a cutting device to follow the path. In addition, the computer memory can be pre-programmed with many standards. The operator at the cutting workstation can enter one of several pre-programmed algorithms via the user interface 216 to perform the task at hand.

The cutting device 220 is configured to receive signals from the computer and can cut the hog carcass accordingly as shown in FIGURE 5. The cutting device 220 may have one or a plurality of cutting implements, such as circular or band saws. Preferably, the cutting device is a high pressure water jet as described in Patent No. 5,931,178 to Pfarr, which is herein incorporated by reference. A plurality of cutting jets may ride on an articulating arm which moves bi-directionally, meaning that the arm can carry the cutting jets horizontally in both a longitudinal and traverse direction to the hog carcass. In addition, the cutting jets may articulate in a vertical direction as well to provide a cutting path along a third axis.

Other alternatives for a cutting station are to provide a series of multiple water jet heads to perform two cutting paths simultaneously. For example, one standard calls for removal of the fatback, while another standard calls for removal of defects below the scribe line. Both tasks can be completed simultaneously with multiple cutting jets. The tasks may also be sequenced if only one cutting jet is available.

Other alternates of the preferred embodiment can include portioning other primals of the carcass. The sequence of steps described in FIGURE 6, may suitably be adapted for any portioning step, provided there are suitable termini of a tissue from which to build a reference. In addition, the method of the preferred embodiment, may be "stacked" on top of one another, meaning that the method is sequentially performed. For example, a primal such as the ham is first portioned using the method of the present invention, followed by portioning the shoulder, again by the method of the present invention, followed by the ribs, loin and fatback, sequentially portioned, and using the method of the present invention. It is also to be understood that the method can be used in combination with other portioning methods which do not rely on a reference line. The benefit of the present invention is that it may be incorporated into existing facilities, since much of the equipment may be located on site already.

While the preferred embodiment of the invention has been illustrated and described, it will be appreciated that various changes can be made therein without departing from the spirit and scope of the invention.

## Claims

1. A method for trimming a foodstuff having tissue with termini, comprising:
scanning the foodstuff,
detecting the termini of the tissue
generating a line with the aid of a computer by using the termini as points on the line, and
trimming the foodstuff with reference to the line.

2. The method of Claim 1, wherein the foodstuff is a pork belly.

3. The method of Claim 2, wherein the tissue comprises fat and lean tissue.

4. The method of Claim 3, wherein the line is a scribe line.

5. A method for trimming a foodstuff having fat and lean tissue, comprising:
scanning the foodstuff;
discerning the fat from the lean tissue;
building a reference from the ends of the lean tissue to guide a trimming device; and
trimming the foodstuff according to the reference.

6. The method of Claim 5, wherein the foodstuff is a pork belly.

7. The method of Claim 5, wherein the reference is a scribe line.

8. The method of Claim 5, wherein the device is a high-pressure water jet.

9. The method of Claim 5, wherein the scanning step is carried out with an optical camera.

10. A method for trimming a foodstuff having fat and lean tissue, comprising:
scanning the foodstuff,
detecting terminus points on the lean tissue,
generating a line with the points, and
trimming the foodstuff with reference to the generated line.

11. The method of Claim 10, wherein the foodstuff is a pork belly.

12. The method of Claim 10, wherein the line is a scribe line.

13. The method of Claim 10, wherein the step of trimming is carried out with a high-pressure water jet.

14. The method of Claim 10, wherein the step of scanning is carried out with an optical camera.

15. The method of Claim 10, wherein the steps of detecting and generating is carried out with a computer.

16. A method for trimming a pork belly having ribs, comprising:
removing the ribs from the pork belly to expose fat lines and lean tissue, and
trimming the pork belly with reference to the ends of the alternating lean and fat lines.

17. A method for portioning a side of a hog carcass having ribs, fatback, loin and belly primals, comprising:
removing the ribs from the carcass to expose fat and lean tissue,
portioning the fatback with reference to a computer generated scribe line, and
trimming the belly with reference to the computer generated scribe line.
